Europäisches Patentamt

European Patent Office

Office européen des brevets

(11) Publication number: **0 396 334**
**A1**

## (12) EUROPEAN PATENT APPLICATION

(21) Application number: 90304545.8

(51) Int. Cl.5: **A61L 9/12, B65D 83/00**

(22) Date of filing: 26.04.90

(30) Priority: **05.05.89 GB 8910427**

(43) Date of publication of application:
**07.11.90 Bulletin 90/45**

(84) Designated Contracting States:
**AT BE CH DE DK ES FR GB GR IT LI LU NL SE**

(71) Applicant: **LOGOBUGS LIMITED**
**18 Guilford Road**
**St. Albans Hertfordshire AL1 5JY(GB)**

(72) Inventor: **Roberts, John Lloyd**
**17 The Four Tubs**
**Bushey Heath, Watford, Hertfordshire(GB)**

(74) Representative: **Shaw, Laurence**
**George House George Road**
**Edgbaston Birmingham B15 1PG(GB)**

(54) Airfreshener.

(57) An airfreshener in the shape of a real or imaginary animal comprises a fluffy body portion (1) and a base portion (4) in the shape of feet or a pair of boots or shoes and having a perforated area (10). A vaporific material (9) preferably in tablet form, is housed within the base portion (4) and is allowed to evaporate slowly over a period of months through the perforated area (10).

FIG.1

## AIRFRESHENER

The invention relates to an airfreshener, primarily but not exclusively intended for use in vehicles, e.g. cars, to clear the fumes caused by smokers and other unpleasant smells. Such devices are well known. One class of device comprises a flat plastics body on a backing card and includes a slow release vaporific deodorant substance. Such devices are not very attractive visually and appear rather lifeless. Another class comprises a fluffy body portion housing a capsule of gelatine or the like, and containing the deodorant substance. The body usually has the appearance of a bug or a real or imaginary animal, with eyes, ears, feet, antennae, etc. and may be attached to a flag or banner on which is printed an advertising message or an amusing slogan. Such bugs are visually appealing, but it can be awkward to release the deodorant from the capsule. This is usually done by perforating the capsule wall and squeezing to release at least some of the container substance. The volume of deodorant which can be contained in the capsule is small and the formulation is such that it tends to evaporate quickly so that the deodorant effect is of limited duration.

It is one object of the invention to provide an airfreshener in the shape of a real or imaginary animal incorporating a vaporific substance which is easy to release when the deodorant effect is required and which will allow the deodorant to be released slowly and be effective over a period of months.

According to the invention in one aspect there is provided an airfreshener in the shape of a real or imaginary animal or a part thereof, the airfreshener comprising a body portion, a base portion, and containing a supply of vaporific material and comprising means for releasing the vaporific material to freshen the ambient air characterised in that the base portion defines a compartment to receive the supply of vaporific material, the material being presented in solid form, a wall of the compartment being perforated so that, in use, vapour may escape from the compartment.

Preferably, the base portion comprises a shell e.g. of vacuum formed plastics resembling the upper part of a foot or boot or shoe, and the perforated wall is in the sole thereof.

Preferably releasable sealing means are present over the perforated wall portion to prevent release of vapour before use and in transport.

In a much preferred feature, the vaporific material is present in a porous carrier therefor, and the carrier is in block or granular form.

In another aspect an airfreshener as defined is characterised by a pad attached to an unperforated portion of the sole pad being adapted to locate the airfreshener on a substrate and being sufficiently thick such that, in use, air currents can pass between the perforated portion and the facing substrate to allow vaporific material to be released to the atmosphere.

In order that the invention may be well understood it will now be described, by way of example only, with reference to the accompanying diagrammatic drawings, in which:

Figure 1 is a front elevation of an airfreshener of the invention in the form of a bug having boots which are shown in section;

Figure 2 is an underneath plan view of the bug shown in Figure 1;

Figure 3 is a side view of the bug of Figures 1 and 2, including a longitudinal sectional view through one of the boots, and

Figure 4 is front elevation of another embodiment of the invention with a cross sectional view taken through the boots.

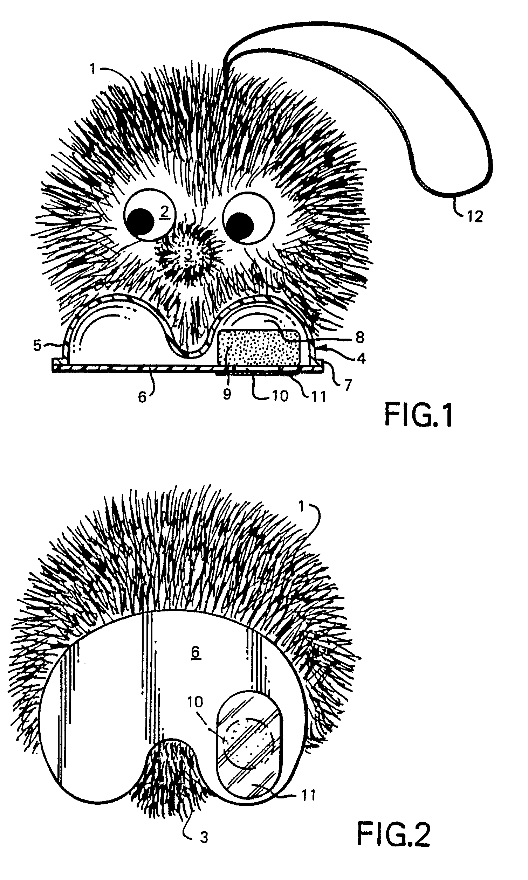

The airfreshener shown in Figures 1 to 3 is of bug like form comprising a fluffy pom-like body portion 1 having eyes 2 and a nose 3. The body 1 is secured to a base portion 4 in the shape of, as shown, a pair of boots 4. The base portion 4 comprises a one piece vacuum formed shell 5 making up all of the boot apart from the underside, i.e. the sole 6 which is formed of a separate flat element. The shell 5 is formed of a suitable plastics, e.g. polypropylene, polyethylene, acrylonitrile butadiene styrene, and the sole 6 may be cardboard, plastics or the like. The shell 5 has a peripheral edge or rim 7 to which the sole 6 is adhered, e.g. using an adhesive. The shell 5 and sole 6 define a compartment 8 which houses a vaporific substance 9 preferably presented in a porous carrier, e.g. chalk or plastics. As shown, a tablet of the carrier 9 is received within one of the boots. In other embodiments (not shown) granules of the carrier are simply present on the floor of the compartment defined by the sole 6. A single hole or perforation 10 is present in the sole and this is releasably sealed by a tear-off strip 11. The bug has a hanging means comprising a loop 12 by which it may be suspended from, e.g. the stem of the interior mirror of a car.

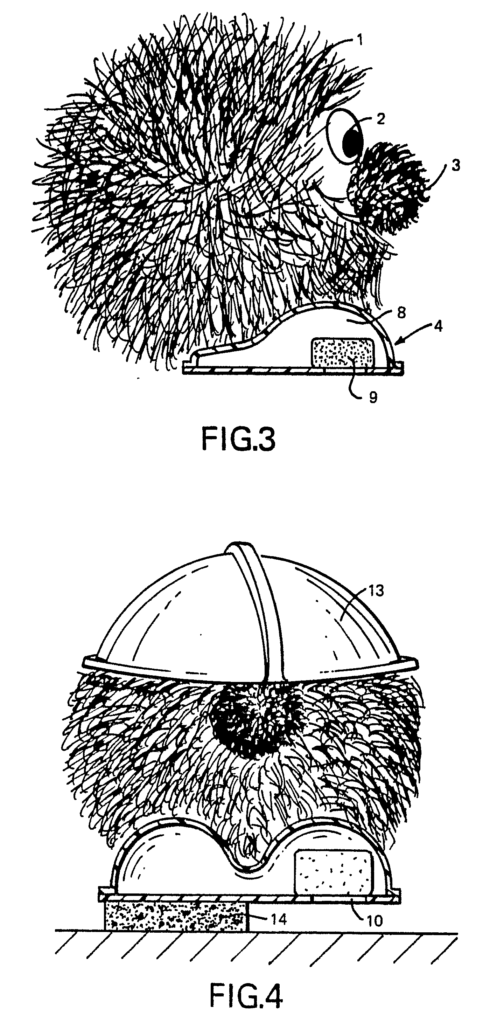

The airfreshener shown in Figure 4 is generally similar to that shown in Figures 1 to 3, but has a hat 13 and no hanging loop. The bug is designed to be adhered to a surface within the vehicle, e.g. the dashboard. A relatively thick pad 14 of e.g. a plastics foam about 3 mm thick is secured to an unperforated portion of the sole 6. In use, the presence of the relatively thick pad 14 allows air to circulate underneath the sole between the perforated portion and the underlying substrate and so allows the deodorant to be released to the atmosphere.

Both bugs are made by adhering the body portion 1 to the vacuum formed shell 5. The tablet 9 of the vaporific substance is placed in the shell 5 with the bug in the inverted condition, and the sole 6 applied to the rim 7, the perforation 10 being sealed by the strip 11.

The bug is then transported to a retail outlet, e.g. a petrol station shop. The purchaser removes the strip 11 to release the vapour to the atmosphere in the car, and then either hangs the bug by the loop or adheres it to a suitable surface, as appropriate. The vapour is released slowly over a period of months. The bug is an attractive item arousing interest and amusement and the deodorant is made available in a simple way. The deodorant may fill the entire compartment or only a portion thereof.

The invention is not limited to the embodiment shown. For example, the loop 10 may be omitted and the outer surface of the sole 6 may be provided with a contact adhesive. Each foot may be moulded separately or only one boot or shoe may be provided. The perforation may be present in an uppermost portion of the boot.

## Claims

1. An airfreshener in the shape of a real or imaginary animal or a part thereof, the airfreshener comprising a body portion (1), a base portion (4), and containing a supply of vaporific material (9) and comprising means for releasing the vapour to freshen the ambient air, characterised in that the base portion defines a compartment (8) to receive the supply of vaporific material (9), the material being presented in solid form, a wall (6) of the compartment being perforated (10) so that, in use, vapour may escape from the compartment (8).

2. An airfreshener according to Claim 1 characterised in that the vaporific material (9) comprises a vaporific liquid in a porous solid carrier therefor.

3. An airfreshener according to Claim 2 characterised in that the carrier (9) is chalk or porous plastics.

4. An airfreshener according to Claim 2 or 3 characterised in that the carrier (9) is in block or granular form.

5. An airfreshener according to any preceding Claim characterised in that the base portion (4) comprises a shell (5) resembling the upper part of a foot, boot or shoe, and the perforated wall (6, 10) is in the sole thereof.

6. An airfreshener according to Claim 5 characterised in that releasable sealing means (11) covers the perforated wall portion (6,10) to prevent release of vapour before use and in transport.

7. An airfreshener according to any preceding Claim characterised by hanging means (12) by which the air freshener may be hung within the interior, e.g. of an automobile or like vehicle.

8. An airfreshener according to Claim 6 characterised by a pad (14) attached to an unperforated portion of the sole (6) pad being adapted to locate the airfreshener on a substrate and being sufficiently thick such that, in use, air currents can pass between the perforated portion (10) and the facing substrate to allow vaporific material to be released to the atmosphere.

9. An airfreshener according to Claim 8 characterised in that the pad (14) is secured to the underside of the sole (6).

FIG.1

FIG.2

FIG.3

FIG.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 125 899  (LOGOBUGS LTD)<br>* Claims 1-5 *<br>--- | 1 | A 61 L   9/12<br>B 65 D   83/00 |
| A | GB-A-2 123 352  (HAKUGEN CO., LTD)<br>* Page 1, lines 18-37 *<br>--- | 1-9 | |
| A | FR-A-2 534 882  (SEYTIER ANDRE)<br>--- | | |
| A | US-A-4 814 212  (DONALD SPECTOR)<br>--- | | |
| A | US-A-4 419 395  (TERUTAKA SUGIMOTO)<br>--- | | |
| A | US-A-4 201 289  (FRANK J. MACK et al.)<br>----- | | |

TECHNICAL FIELDS
SEARCHED (Int. Cl.5)

A 61 L

The present search report has been drawn up for all claims

| Place of search | Date of completion of the search | Examiner |
|---|---|---|
| THE HAGUE | 31-07-1990 | ESPINOSA Y CARRETERO M. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
............................................................
& : member of the same patent family, corresponding
document

EPO FORM 1503 03.82 (P0401)